(19) Europäisches Patentamt
European Patent Office
Office européen des brevets

(11) **EP 1 261 279 B1**

(12) **FASCICULE DE BREVET EUROPEEN**

(45) Date de publication et mention
de la délivrance du brevet:
**05.09.2007 Bulletin 2007/36**

(51) Int Cl.:
**A61B 5/103** [(2006.01)]

(21) Numéro de dépôt: **01907706.4**

(22) Date de dépôt: **05.02.2001**

(86) Numéro de dépôt international:
**PCT/FR2001/000340**

(87) Numéro de publication internationale:
**WO 2001/056470 (09.08.2001 Gazette 2001/32)**

(54) **PROCEDE D'ANALYSE D'IRREGULARITES DE LOCOMOTION HUMAINE**

ANALYSEVERFAHREN VON UNREGELMÄSSIGKEITEN IN MENSCHLICHER FORTBEWEGUNG

METHOD FOR ANALYSING IRREGULARITIES IN HUMAN LOCOMOTION

(84) Etats contractants désignés:
**AT BE CH CY DE DK ES FI FR GB GR IE IT LI LU MC NL PT SE TR**

(30) Priorité: **04.02.2000 FR 0001461**

(43) Date de publication de la demande:
**04.12.2002 Bulletin 2002/49**

(73) Titulaire: **INSTITUT NATIONAL DE LA RECHERCHE AGRONOMIQUE**
**75341 Paris Cedex 07 (FR)**

(72) Inventeurs:
• **BARREY, Eric**
  **F-77630 Barbizon (FR)**
• **AUVINET, Bernard**
  **F-53000 Laval (FR)**

(74) Mandataire: **Catherine, Alain**
**Cabinet Harlé & Phélip**
**7, rue de Madrid**
**75008 Paris (FR)**

(56) Documents cités:
• **SEKINE M. ET AL: "classification of acceleration waveform in a continuous walking record" PROCEEDINGS OF THE 20TH ANNUAL INTERNATIONAL CONFERENCE OF THE IEEE ENGINEERING IN MEDICINE AND BIOLOGY SOCIETY, vol. 20, no. 3, 29 octobre 1998 (1998-10-29) - 1 novembre 1998 (1998-11-01), pages 1523-1526, XP000864114 Piscataway, US**
• **DATABASE INSPEC [en ligne] INSTITUTE OF ELECTRICAL ENGINEERS, STEVENAGE, GB; TAMURA T ET AL: "Classification of acceleration waveforms during walking by wavelet transform" Database accession no. 5826646 XP000864115 & BIOSIGNAL INTERPRETATION II, KANAGAWA, JAPAN, SEPT. 1996, vol. 36, no. 4-5, pages 356-359, Methods of Information in Medicine, Dec. 1997, F.K. Schattauer Verlagsgesellschaft, Germany ISSN: 0026-1270**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention se rapporte à un procédé d'analyse d'irrégularités de locomotion humaine.

L'invention s'applique notamment à l'analyse de la marche humaine dans la pratique médicale et paramédicale, en particulier pour étudier la dégradation de la marche et effectuer des prédictions de chute pour mettre en place des mesures de prévention, par exemple chez des personnes âgées. L'invention s'applique également à des mesures de claudication faisant suite à des traumatismes ou des traitements médicaux ou chirurgicaux. Un autre domaine d'application est celui de la marche ou de la course sportive.

Deux familles de techniques complémentaires ont été développées pour étudier les mouvements du corps humain et leur mécanique et ont ainsi permis d'accroître considérablement les connaissances sur la physiologie de la locomotion humaine:

- des méthodes cinématiques, qui mesurent des déplacements des parties du corps et des angles articulaires, au moyen d'images cinématographiques ou vidéo et
- des méthodes dynamiques, qui mesurent des forces ou des accélérations agissant sur des parties du corps.

Les méthodes cinématiques sont descriptives et apportent de nombreux détails sur les mouvements de chaque segment corporel, mais sont lourdes à mettre en oeuvre. Les méthodes dynamiques sont plus explicatives, car elles donnent des informations sur les actions mécaniques à l'origine du mouvement. Elles apportent ainsi une information plus synthétique qui reste néanmoins très fine.

Plusieurs instruments de mesure connus, mis en oeuvre dans les méthodes dynamiques (aussi appelées cinétiques) sont des semelles baropodométriques, des plates-formes de force et des accéléromètres. Les semelles baropodométriques peuvent apporter des données utiles à un clinicien, mais ne constituent pas un instrument de mesure des forces. Les plates-formes de force donnent quant à elles des informations fiables et précises, mais le matériel est encombrant et coûteux. De plus, leurs dimensions n'autorisent qu'un seul appui, ce qui donne une information tronquée et ne permet pas une analyse de variabilité de la marche.

Les accéléromètres, constitués de capteurs sensibles aux variations instantanées de vitesse n'ont pas les inconvénients ci-dessus et permettent d'obtenir des résultats précis et fiables. On se reportera en particulier à l'article de Bernard AUVINET, Denis CHALEIL et Eric BARREY, « Analyse de la marche humaine dans la pratique hospitalière par une méthode accélérométrique », REV. RHUM. 1999, 66 (7-9), pp. 447-457 (édition française) et pp. 389-397 (édition anglaise).

Mesurer les accélérations présente l'avantage de fournir des informations sensibles et détaillées sur les forces engendrant des mouvements et peut être simple à mettre en oeuvre. De plus, le conditionnement et le traitement de données est économique par rapport à l'utilisation d'images. Par ailleurs, des mesures d'accélération permettent de retrouver toutes les caractéristiques cinétiques et cinématiques d'un mouvement.

Le traitement des résultats obtenus, pour détecter et analyser d'éventuelles irrégularités de locomotion, repose généralement sur des transformations de Fourier. L'article d'AUVINET et al. mentionné ci-dessus propose également des calculs fondés sur la fonction d'autocorrélation de signaux (étude de la symétrie et de la régularité des foulées), complétée par une transformation z de Fischer pour parvenir à des distributions gaussiennes.

Un inconvénient des analyses fréquentielles (telles qu'effectuées par des transformations de Fourier rapides) est qu'elles nécessitent un nombre minimal de points pour avoir une information suffisamment précise en fréquence et en énergie. De plus, elles n'autorisent pas une localisation dans le temps d'un événement particulier provoquant un changement fréquentiel rapide du signal d'accélération. Il n'est donc pas possible de détecter un faux pas isolé parmi d'autres pas normaux. L'analyse fréquentielle est également mal adaptée à des irrégularités temporelles du cycle locomoteur, qui ne sont pas stationnaires mais ont des propriétés chaotiques (imprédictibles à courts et longs termes).

En ce qui concerne les méthodes de calcul fondées sur des fonctions d'autocorrélation, elles permettent une très bonne détection d'asymétries dynamiques périodiques mais sont peu performantes pour détecter des irrégularités qui ne sont ni stationnaires, ni périodiques. Elles ne permettent pas non plus d'identifier un événement isolé comme une faute de marche sportive ou un faux pas.

Par ailleurs, plusieurs techniques spécifiques ont été développées pour étudier des mouvements complexes dans la locomotion équine, reposant également sur des mesures d'accélérations. Cependant, les mouvement étudiés consistent en des sauts de chevaux de sport ou des transitions d'allure de chevaux de dressage (passage du trop au galop ou inversement), donc en des mouvements transitoires. Ces techniques ne s'appliquent donc pas à des mouvements à allure stabilisée, tels que la marche ou la course humaine.

Un procédé de classification des mouvements basé sur les signaux fournis par un dispositif accéléromètre triaxial avec un enregistrement des signaux réalisé de manière continue dans le temps, dans lequel on analyse les signaux selon une transformée en ondelettes de Coiflet, est divulgué dans le document de Sekine et al : "Classification of accélération waveform in a continuous walking record". Proceedings of the 20th Annual conference of the IEEE in Medicine and Biology Society, Vol. 20, No3, 1988, pages 1523-1526.

La présente invention concerne un procédé d'analyse d'irrégularités de locomotion humaine reposant sur des mesures d'accélération, qui permet de détecter des cycles locomoteurs anormaux et irréguliers dans le temps.

Le procédé de l'invention ne requiert pas de propriétés mathématiques a priori des signaux étudiés, telles que celles nécessitées pour des transformations de Fourier (stationarité, périodicité).

De plus, le procédé de l'invention peut autoriser une analyse rapide et simple des résultats obtenus.

L'invention a également pour objet un procédé d'analyse pouvant donner des informations complémentaires quantitatives sur le manque de régularité dynamique de cycles de marche ou de course.

A cet effet, l'invention s'applique à un procédé d'analyse d'irrégularités de locomotion humaine, dans lequel on utilise des mesures d'accélération obtenues pendant au moins un déplacement contrôlé à allure stabilisée d'un être humain, au moyen d'au moins un accéléromètre mesurant en fonction du temps au moins une accélération selon au moins une direction, en détectant et en analysant d'éventuelles irrégularités de locomotion à partir de mesures de référence.

Selon l'invention, on soumet les accélérations mesurées à au moins une transformation en ondelettes et on se sert de la transformée en ondelettes obtenue pour détecter et/ou analyser les irrégularités.

Par « allure stabilisée », on entend un mouvement répétitif, approximativement périodique (tel que la marche ou la course), par opposition à un mouvement transitoire.

De manière surprenante, la transformation en ondelettes permet de repérer des anomalies au cours du temps pour une telle allure stabilisée. Même un seul faux pas isolé est détectable parmi des pas normaux. Ce procédé est ainsi apte à détecter des irrégularités très brèves, contrairement aux méthodes connues reposant sur des transformations de Fourier.

Préférentiellement, on visualise un spectre de la transformée en ondelettes en trois dimensions respectivement de temps, de fréquence et de module d'énergie spectrale de la transformée en ondelettes pour détecter et analyser les irrégularités.

On représente alors avantageusement le module d'énergie spectrale de la transformée en ondelettes par des contours colorés ou par un gradient de couleurs. De plus, on utilise avantageusement une échelle linéaire pour le temps et logarithmique pour la fréquence.

La visualisation directe de l'ondelette offre une grande simplicité de lecture et permet d'identifier directement des irrégularités par lecture graphique.

Préférentiellement, la transformation en ondelettes est continue, cette transformation étant avantageusement une transformation en ondelettes de Morlet. Ces ondelettes, symétriques, sont bien adaptées à des analyses de basses fréquences, telles que les accélérations corporelles à proximité du centre de gravité. Elles sont données par la formule mathématique (fonction $\psi$ de la variable x):

$$\psi(x) = k.\exp(-x^2/2) . \cos(\omega_o x),$$

où k est une constante de normalisation et $\omega_o$ est la pulsation de l'ondelette considérée, préférentiellement comprise entre 5 et 6 inclus. On décompose une fonction d'accélération Acc dépendant du temps t sur une famille d'ondelettes de ce type par la formule

$$C(a, b) = \int_{-\infty}^{+\infty} Acc(t).\psi(at+b)dt,$$

où:

- les coefficients C donnent les projections de la fonction Acc sur les ondelettes,
- le paramètre *a* est un paramètre d'échelle (modification de l'étirement des ondelettes) et
- le paramètre *b* est un paramètre de translation (translation dans le temps des ondelettes).

Dans une forme de mise en oeuvre préférée, la transformée en ondelettes ayant une énergie spectrale en fonction du temps, on définit une bande de basse fréquence dans laquelle est principalement concentrée l'énergie spectrale pour une locomotion normale et on détecte et/ou on analyse lesdites irrégularités en utilisant l'énergie spectrale en dehors de cette bande. La bande de fréquence est préférentiellement comprise entre 1 Hz et 4 Hz.

L'utilisation de cette bande rend notamment possible deux types d'analyse qui peuvent être complémentaires:

- l'identification de zones de concentrations d'énergie spectrale hors bande, et
- la quantification d'énergie spectrale en dehors de cette bande.

Ainsi, avantageusement, on identifie et/ou on analyse les irrégularités en repérant et/ou en étudiant des pics d'énergie spectrale en dehors de la bande.

Ainsi, on peut observer pour une marche pathologique des pics de haute fréquence ayant une densité énergétique significative dépassant la limite supérieure de la bande de fréquence (par exemple 4 Hz), ces pics étant plus ou moins périodiques et réguliers dans le temps et en fréquence. Des irrégularités graphiques de la forme de ces pics sont révélatrices d'altérations de la dynamique et de la régularité de la marche.

De plus, on calcule avantageusement un degré d'irrégularités de locomotion en rapportant l'énergie spectrale marginale en dehors de la bande, à l'énergie spectrale totale.

Ainsi, pour une marche pathologique, on peut considérer que l'énergie marginale au-dessus de 4 Hz est supérieure à 6%.

Dans un autre type avantageux d'analyse reposant sur la bande de basse fréquence, on mesure les valeurs localisées d'énergie spectrale qui dépassent la bande, par exemple dans le cas d'une marche très altérée avec des motifs de haute fréquence et de forme et d'énergie très variées.

Les analyses par transformation en ondelettes sont avantageusement associées à d'autres méthodes donnant des informations complémentaires dans le procédé d'analyse d'irrégularités de locomotion humaine. De telles informations sont combinées avec les résultats obtenus au moyen des transformations en ondelettes, pour spécifier le mode d'utilisation des ondelettes, interpréter les résultats obtenus par ces ondelettes et/ou compléter les résultats extraits des analyses par ondelettes.

Ainsi, dans une forme avantageuse de mise en oeuvre, les procédés d'analyse selon l'une quelconque des accélérations sont au moins au nombre de deux et pour détecter et/ou analyser les irrégularités, on utilise aussi au moins un vectogramme (dit « papillon frontal ») représentant l'intensité d'une des accélérations mesurées en fonction de l'intensité d'une autre des accélérations mesurées. On peut ainsi identifier visuellement des dérives chaotiques par rapport à un régime stationnaire et périodique.

Les accélérations utilisées sont préférentiellement une accélération verticale et une accélération latérale du sujet.

Dans un mode de mise en oeuvre préféré, pour détecter et/ou analyser les irrégularités, on utilise aussi au moins une représentation dans un espace des phases, donnant l'intensité d'au moins une des accélérations en fonction de l'intensité de la dérivée temporelle de cette accélération ou en fonction de cette accélération décalée dans le temps d'un délai prédéterminé.

On peut ainsi identifier visuellement l'éloignement d'un régime stationnaire et périodique vers un régime chaotique. Avantageusement, l'accélération étudiée est une accélération verticale.

Dans cette mise en oeuvre préférée, on calcule avantageusement au moins un coefficient de Lyapunov de la représentation dans l'espace des phases, préférentiellement le coefficient de Lyapunov maximal. On peut ainsi quantifier des irrégularités dynamiques de la locomotion, un tel coefficient mesurant la vitesse de divergence des orbites des signaux d'accélération dans l'espace des phases.

Ainsi, le coefficient de Lyapunov maximal, calculé sur un signal accélérométrique cranio-caudal mesuré au niveau du centre de gravité d'un sujet, quantifie globalement un manque de régularité dynamique des cycles de marche. Il permet de détecter des fluctuations temporelles et dynamiques de coordination en relation avec un risque de chute. Cette mesure globale de régularité de la marche a un intérêt clinique pour une prédiction du risque de chute d'un sujet âgé. La marche peut ainsi être considérée comme pathologique lorsque le coefficient de Lyapunov utilisé est supérieur à une valeur critique.

Ainsi, avantageusement, on considère que se produit un désordre de coordination si le coefficient de Lyapunov maximal est supérieur à 0,4.

Le dispositif utilisé pour les accéléromètres et les mesures d'accélération est préférentiellement conforme à ce qui est exposé dans l'article d'AUVINET et al. cité plus haut. En particulier, le procédé d'analyse admet avantageusement les caractéristiques suivantes, considérées séparément ou selon toutes leurs combinaisons techniquement possibles:

- les accélérations sont mesurées au moyen d'un capteur accélérométrique comprenant deux accéléromètres disposés selon des axes perpendiculaires;
- les accéléromètres sont incorporés dans une ceinture semi-élastique fixée à la taille du sujet, de telle manière qu'ils s'appliquent en région lombaire médiane, en regard de l'espace intervertébral L3-L4;
- les accéléromètres sont disposés à proximité du centre de gravité du sujet (ce dernier se localisant chez l'homme en position debout au repos en avant de la deuxième vertèbre sacrée);
- les accélérations sont mesurées selon des axes cranio-caudaux et latéraux du sujet, ci-après désignés respectivement par axes verticaux et latéraux;

- les mesures d'accélération sont enregistrées au moyen d'un enregistreur portatif;
- on complète les résultats obtenus au moyen de la transformation en ondelettes, par l'obtention de la fréquence des foulées, de la symétrie et de la régularité des pas, les variables symétrie et régularité étant avantageusement soumises à une transformation z de Fischer;
- les déplacements contrôlés à allure stabilisé consistent en une marche libre à la vitesse de confort du sujet sur une distance comprise entre 25 et 50 m et préférentiellement égale à 40 m, avantageusement aller et retour.

De plus, on met avantageusement en oeuvre les caractéristiques suivantes, séparément ou en combinaison:

- les accélérations sont aussi mesurées selon un troisième accéléromètre, les trois accéléromètres étant disposés selon des axes perpendiculaires correspondant respectivement aux axes cranio-caudal, latéral et longitudinal (antéro-postérieur) du sujet;
- pour l'analyse de mouvements sportifs, les accélérations mesurées sont des accélérations cranio-caudales et longitudinales (plan sagittal du sujet);
- pour l'analyse de mouvements sportifs, les mesures sont pratiquées au cours de tests d'effort sur piste ou sur tapis roulant;
- on met également en oeuvre une analyse fréquentielle de Fourier complémentaire dans le procédé d'analyse, en calculant:

  - l'énergie totale du spectre (celle-ci diminue pour une marche pathologique),
  - l'énergie relative de la fréquence fondamentale, qui correspond à la fréquence du pas (celle-ci diminue et se distribue vers des harmoniques de plus hautes fréquences pour une marche pathologique) et/ou
  - la pente du spectre, calculée par une équation de régression linéaire sur une représentation semi-logarithmique de l'énergie spectrale au carré en fonction de la fréquence (cette pente est d'autant plus négative que la marche est altérée dans sa régularité temporelle et dynamique).

De plus, on a avantageusement recours à la fois à un capteur comprenant les accéléromètres, à un enregistreur et à un appareil de marquage d'événements, prévu pour marquer des événements sur des signaux d'accélération enregistrés et/ou synchroniser les enregistrements d'accélération avec d'autres appareils de mesure (caméra vidéo ou cinématographique, plate-forme de force, cellule de chronométrage, appareil EMG, etc.). Cet appareil est avantageusement interposé entre le capteur et l'enregistreur, et avantageusement intégré dans un boîtier d'enregistrement de l'enregistreur.

L'appareil de marquage d'événements a préférentiellement des entrées d'activation constituée par des contacts électriques manuels, optoélectroniques (cellule sensible à un flash de lumière) et/ou électroniques.

Il a préférentiellement des sorties constituées:

- d'un émetteur d'une onde carrée, ayant avantageusement une valeur maximale (saturation) d'une durée comprise entre 10 et 50 ms et préférentiellement de 10 ms pour une fréquence d'acquisition du signal à 100 Hz, avantageusement émise sur les voies du signal d'accélération afin de marquer ce signal sur un point de mesure;
- d'une série de diodes luminescentes (LED) rouges, s'allumant pour donner un signal lumineux visible sur une seule image par une caméra vidéo, avantageusement pendant une durée comprise entre 10 et 50 ms et préférentiellement égale à 10 ms; et/ou
- d'une sortie TTL fournissant avantageusement un signal carré de 5 V pendant une durée comprise entre 10 et 50 ms et préférentiellement égale à 10 ms.

L'utilisation d'un tel appareil de marquage d'événements est particulièrement intéressante pour marquer des instants de passage (départ et arrivée d'un test de marche ou d'une course) sur des enregistrement d'accélération. On peut ainsi obtenir un repérage spatial ou cinématique (film vidéo synchronisé) du mouvement enregistré afin de calculer des distances représentatives d'un mouvement sportif.

L'invention concerne aussi les applications du procédé:

- au domaine médical, en particulier pour la détection précoce de désordres neuro-moteurs de sujets âgés (il est alors spécialement intéressant d'utiliser le coefficient de Lyaponov maximal) et
- au domaine sportif, en particulier pour la détection de fautes techniques de marcheurs ou de coureurs sportifs.

Ainsi, le procédé de l'invention peut être avantageusement appliqué à la détection des particularités de la foulée de l'athlète pendant une période de course, tel que le temps d'envol, le temps d'appui, la symétrie des demi-foulées droites et gauches, les forces de propulsion et de freinage droites et gauches et l'indice de fluidité de la course.

Le procédé selon l'invention peut aussi être appliqué pour mesurer et quantifier les contraintes biomécaniques liées à une affection de l'appareil locomoteur, notamment en fonction de la vitesse de la course.

La caractéristique des particularités de la foulée de l'athlète en course selon le procédé de l'invention est utile notamment en vue d'améliorer l'efficacité et les performances du sportif.

La caractérisation des paramètres d'accélération conformément au procédé de l'invention est utile notamment aux fins d'évaluer la tolérance d'une affection de l'appareil locomoteur en fonction des charges de travail.

La présente invention sera illustrée et mieux comprise à l'aide de modes de mise en oeuvre particuliers, aucunement limitatifs, en référence aux figures annexées, sur lesquelles:

La Figure 1 représente un sujet muni d'un capteur d'accélération, en vue de la mise en oeuvre du procédé d'analyse selon l'invention;

La Figure 2 montre un dispositif de mesure, utilisable pour mettre en oeuvre le procédé d'analyse de l'invention et correspondant à celui de la Figure 1;

La Figure 3 montre un agrandissement d'une portion du dispositif de mesure de la Figure 2, sans le capteur;

La Figure 4 représente en vue de face et en coupe le capteur du dispositif de mesure de la Figure 2;

La Figure 5 représente en vue de dessus et en coupe le capteur du dispositif de mesure des Figures 2 et 4;

La Figure 6 montre des courbes d'accélérations verticale et latérale en fonction du temps, synchronisées avec des images d'un sujet, pour une foulée de marche normale;

La Figure 7 montre des courbes d'accélérations verticale et latérale en fonction du temps synchronisées avec des images d'un sujet, pour une foulée de marche pathologique;

La Figure 8 représente des courbes d'évolution dans le temps des accélérations verticale et latérale pour une marche normale d'un sujet;

La Figure 9 montre une image spectrale en trois dimensions d'une transformée en ondelettes de Morlet obtenue à partir de l'accélération verticale de la Figure 8, conformément au procédé d'analyse selon l'invention;

La Figure 10 montre un agrandissement de l'image spectrale de la Figure 9;

La Figure 11 représente une courbe donnant l'évolution en fonction du temps de l'accélération verticale pour une marche pathologique d'un sujet âgé chuteur;

La Figure 12 représente une image spectrale en trois dimensions d'une transformée en ondelettes de Morlet obtenue à partir de l'accélération verticale de la Figure 11;

La Figure 13 est un vectogramme dit « papillon frontal » donnant l'accélération verticale en fonction de l'accélération latérale, correspondant à la marche normale associée aux Figures 8 à 10;

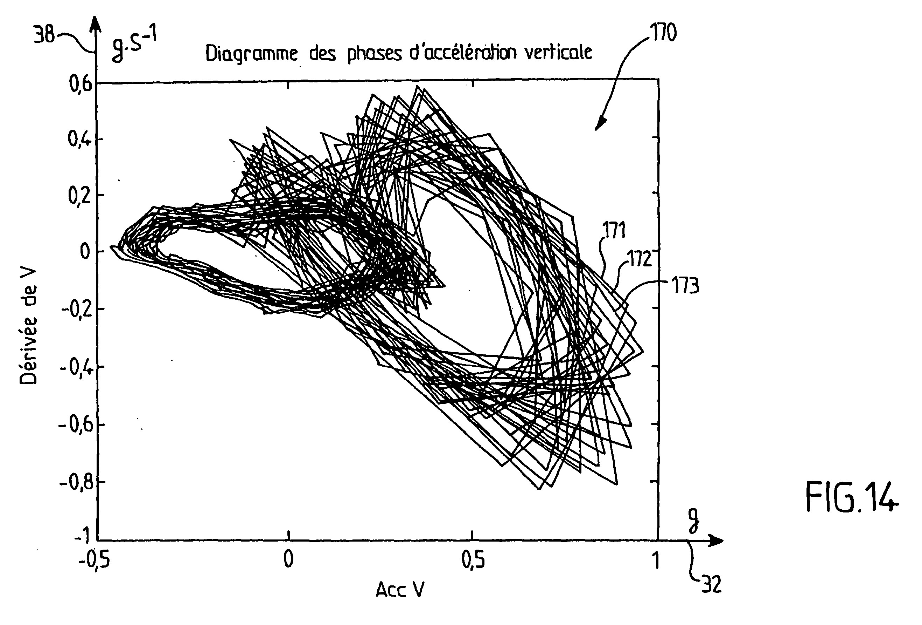

La Figure 14 représente un diagramme des phases d'accélération verticale donnant la dérivée première de l'accélération par rapport au temps en fonction de la série temporelle de cette accélération, pour une marche normale correspondant aux Figures 8 à 10; et

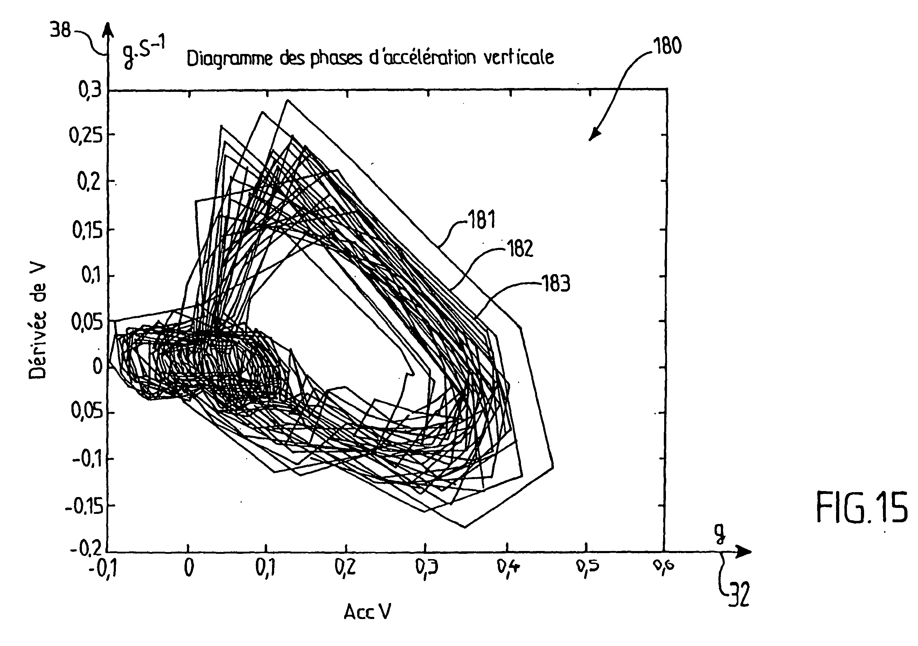

La Figure 15 représente un diagramme des phases d'accélération verticale donnant la dérivée première de l'accélération par rapport au temps en fonction de la série temporelle de cette accélération, pour une marche pathologique correspondant aux Figures 11 et 12.

La figure 16 montre une image spectrale en trois dimensions d'une transformée en ondelettes de Morlet obtenue conformément au procédé de l'invention dans une période de course d'un sportif dont la foulée est très fluide

La figure 17 montre une image spectrale en trois dimensions d'une transformée en ondelettes de Morlet obtenue conformément au procédé de l'invention dans une période de course d'un sportif ayant une forte asymétrie de propulsion et de freinage.

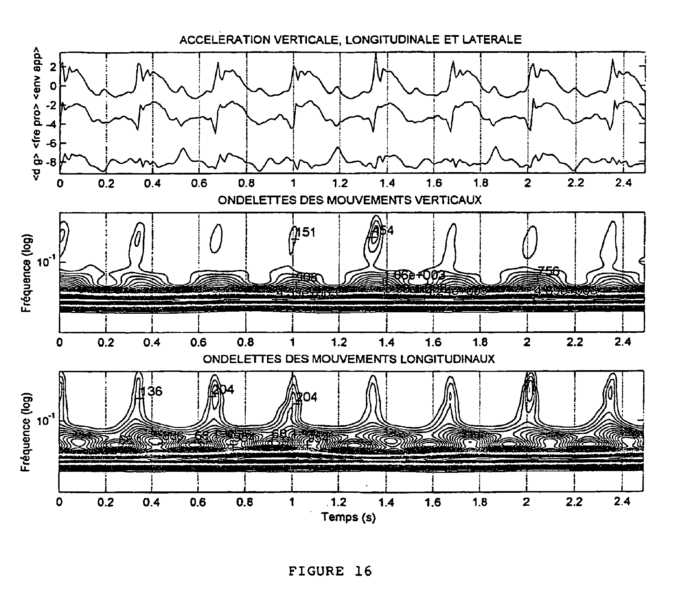

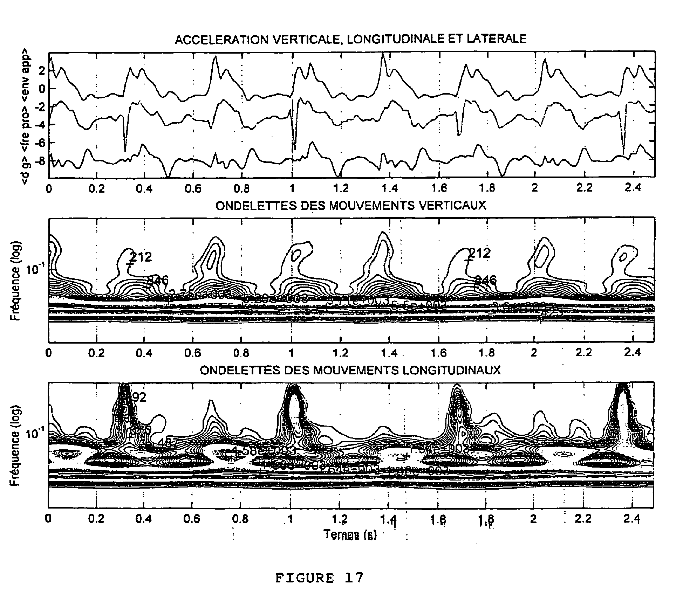

Sur les figures 16 et 17, le graphe supérieur illustre les courbes d'accélération verticale, longitudinale et latérale en fonction du temps exprimé en secondes; le graphe du milieu illustre l'image spectrale de la transformée en ondelettes des mouvements verticaux en fonction du temps; le graphe inférieur illustre l'image spectrale de la transformée en ondelettes des mouvements longitudinaux en fonction du temps exprimé en secondes.

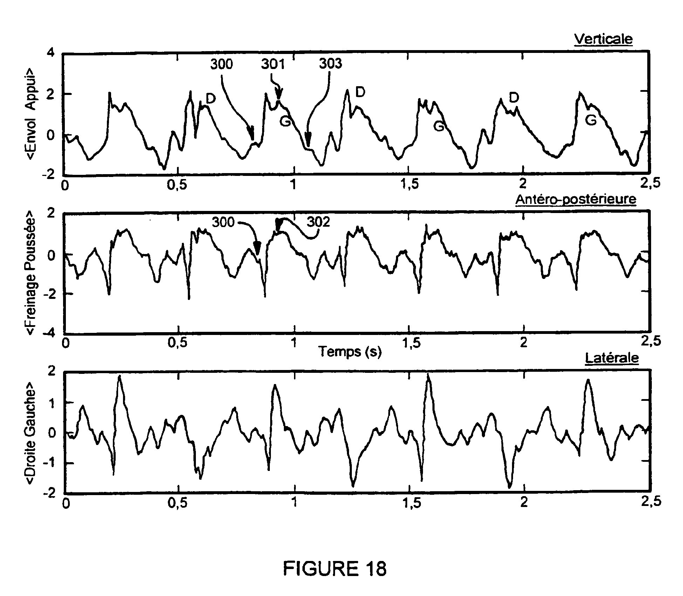

La figure 18 montre des courbes d'accélération verticale, antéro-postérieure et latérale en fonction du temps synchronisées avec des images d'un sujet, pour une foulée de course normale.

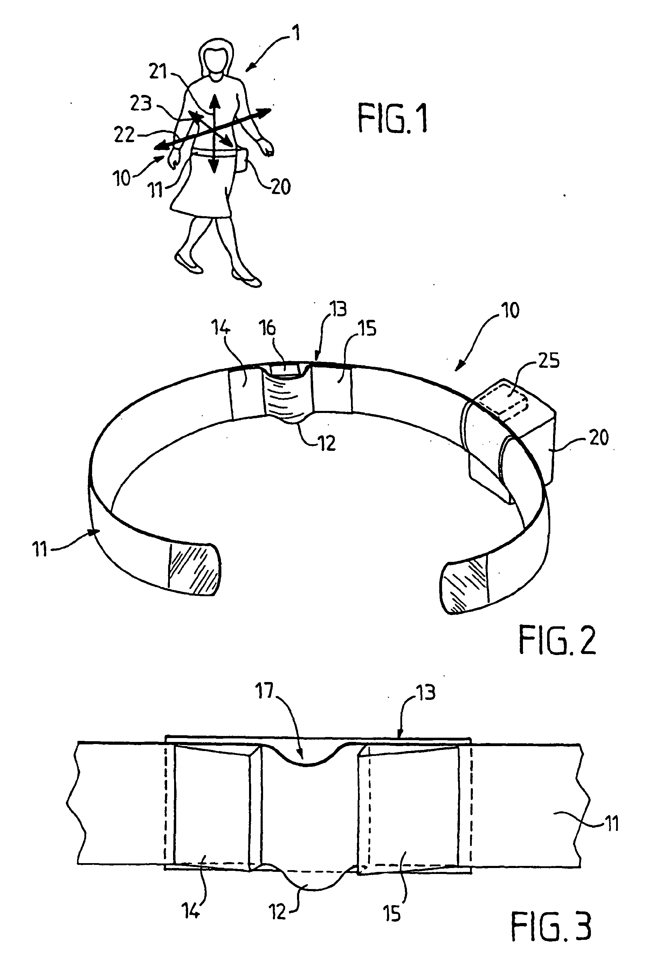

Un dispositif 10 de mesure (Figures 1 et 2) comprend un capteur 16 sensible à des mouvements, connectés à un enregistreur 20 ambulatoire se présentant sous forme de boîtier.

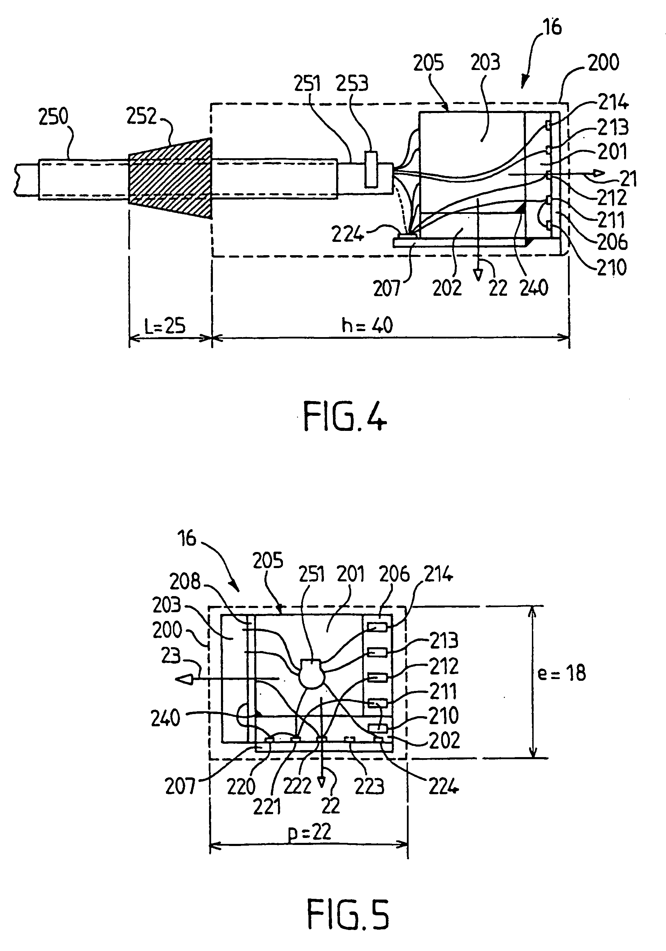

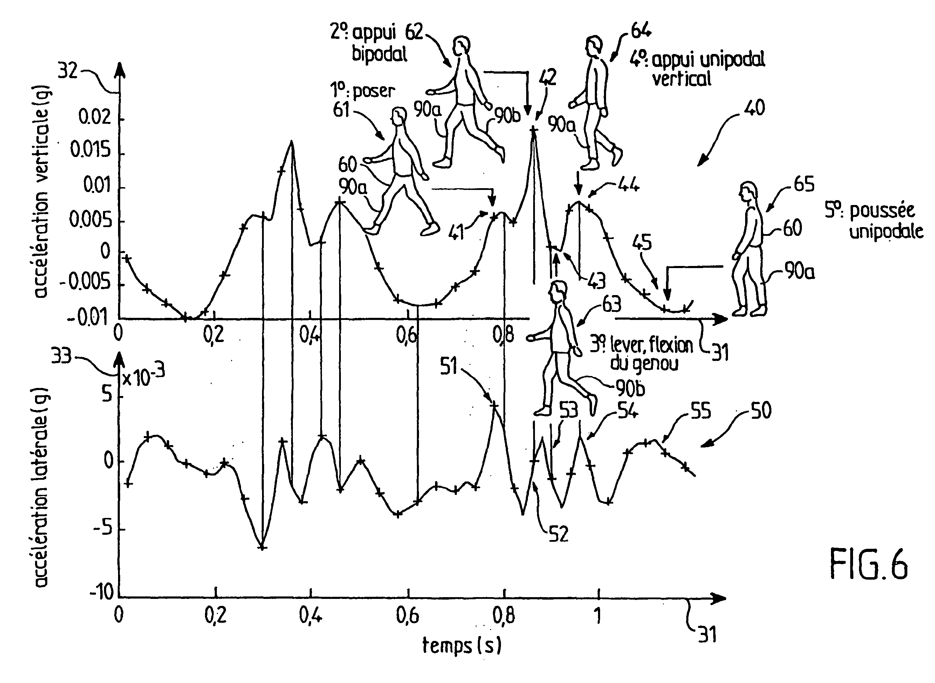

Le capteur 16 est un capteur accélérométrique comprenant (figures 4 et 5) des accéléromètres 201, 202 et 203 disposés perpendiculairement, de manière à détecter des accélérations respectivement dans les directions 21, 22 et 23 perpendiculaires. A titre d'illustration, les trois accéléromètres 201, 202 et 203 s'orientent respectivement selon une première direction 21 cranio-caudale ci-après verticale, selon une deuxième direction 22 latéro-latérale et selon une troisième direction 23 antéro-postérieure (ci-après « longitudinale ») d'un sujet 1. Ces accéléromètres 201-203 sont sensibles à des composantes continues et dynamiques et sont adaptés pour mesurer des mouvements de basses fréquences. Leur fréquence propre est par exemple de 1200 Hz, la gamme de mesure se situant entre $\pm$ 10 g et la sensibilité étant de 5 mV/g à 100 Hz. Ils forment un assemblage 205 cubique, dans lequel leur orthogonalité est assurée au moyen d'équerres 240. Cet assemblage 205 est moulé dans un enrobage 200 polymère parallélépipédique et de petite taille. L'enrobage 200 est isolant, rigide, protecteur et étanche. Il a par exemple une hauteur h (direction 21), une largeur l (direction 22) et une profondeur p (direction 23) valant respectivement 40, 18 et 22 mm.

Chacun des accéléromètres 201, 202 et 203 est muni de cinq bornes de connexion comprenant:

- une borne de masse (bornes 210 et 220 respectivement des accéléromètres 201 et 202),
- une borne d'alimentation négative connectée à la borne de masse (bornes 211 et 221 respectivement des accéléromètres 201 et 202),
- une borne d'alimentation positive (bornes 212 et 222 respectivement des accéléromètres 201 et 202),
- une borne de signal négatif (bornes 213 et 223 respectivement des accéléromètres 201 et 202), et
- une borne de signal positif (bornes 214 et 224 respectivement des accéléromètres 201 et 202).

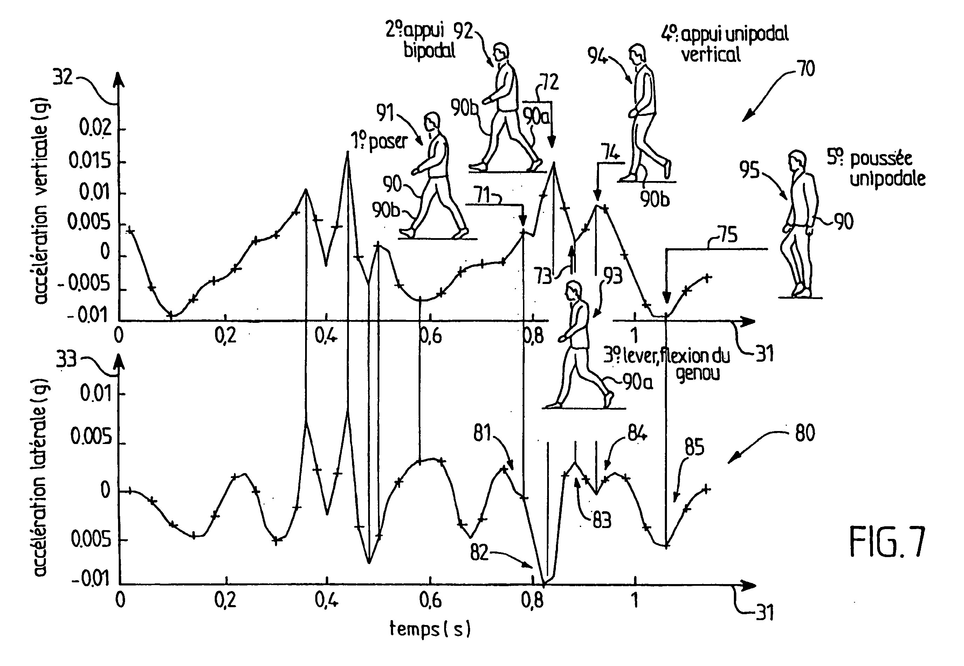

Les bornes et les accéléromètres 201 à 203 reposent respectivement sur des socles en céramique 206 à 208.
Les bornes de masse et les bornes d'alimentation négative des trois accéléromètres 201 à 203 sont reliées entre elles.
Le capteur 16 comprend aussi un fil 251 à douze conducteurs à masse tressée (tresse métallique évitant des parasites), contenant des fils en provenance des bornes d'alimentation et de signal des trois accéléromètres 201 à 203. Ce fil 251, conduisant à l'enregistreur 20, est partiellement entouré d'une gaine 250 semi-rigide thermorétractable pénétrant dans l'enrobage 200. Cette gaine 250 permet d'éviter les cassures du fil. Elle est de plus bordée à l'extérieur de l'enrobage 200 par une partie tronconique 252 formant une seule pièce avec l'enrobage 200, qui évite des torsions et des risques de cassure du fil. La partie tronconique 252 a une longueur L, par exemple égale à 25 mm.
De plus, le fil 251 est muni d'une agrafe 253 prévue pour venir en butée contre la gaine 250 en cas de coulissement du fil 251 dans la gaine 250, de manière à éviter d'arracher les soudures.
Le capteur 16 est incorporé dans une ceinture 11 semi-élastique fixée à la taille du sujet 1, de telle manière que ce capteur 16 s'applique en région lombaire médiane, en regard de l'espace intervertébral L3-L4. Ce positionnement du capteur 16 autorise une bonne stabilité des accéléromètres à proximité du centre de gravité du sujet 1, ce centre de gravité se localisant en avant de la deuxième vertèbre sacrée pour un être humain en position debout au repos.
Le capteur 16 est disposé dans un espace 17 formé par une partie lâche 12 de la ceinture 11 et par un renfort 13 en cuir plaqué contre la ceinture 11, au moyen de cales 14 et 15 en tissu mousse haute densité, disposées de part et d'autre de la partie 12 (Figures 2 et 3). Les cales 14 et 15 permettent de positionner l'enrobage 200 du capteur 16 exactement dans la gouttière vertébrale du sujet 1.
L'enregistreur 20 a par exemple une fréquence d'acquisition de 100 Hz et une autonomie permettant un enregistrement continu pendant 30 minutes. Il est muni d'un filtre passe-bas de fréquence de coupure de 50 Hz pour éviter des phénomènes de repliement. Il admet trois voies perpendiculaires synchronisées. Les mesures d'accélération sont numérisées et codées, par exemple sur 12 bits.
L'enregistreur 20 comprend des moyens de connexion à une unité de traitement, par exemple à un ordinateur de type PC par l'intermédiaire d'un port de communication série à l'aide d'un logiciel de transfert.
Le dispositif de mesure 10 comprend également un appareil de marquage d'événements 25, intercalé électriquement entre le capteur 16 et l'enregistreur 20 et intégré au boîtier de l'enregistreur 20 (Figure 2). Cet appareil 25 a pour fonction de marquer des événements sur des signaux enregistrés et/ou de synchroniser des enregistrements d'accélération avec d'autres appareils de mesure. Il est pourvu d'une cellule sensible à un flash de lumière et est prévu pour saturer le signal accélérométrique, par exemple pendant 0,01 seconde pour une acquisition à 100 Hz au moment où un flash se déclenche.
En fonctionnement, on fait marcher le sujet 1 en ligne droite, par exemple sur une distance de 30 m aller et 30 m retour. On dispose avantageusement de cellules de chronométrage distantes de 30 m aux extrémités du trajet suivi par le sujet, de manière à pouvoir mesurer les vitesses et synchroniser des mesures. Ces cellules de chronométrage forment chacune une barrière infrarouge comprenant un émetteur et un récepteur. Elles sont respectivement reliées à des flashs, qui sont déclenchés lorsque la barrière infrarouge est traversée par le sujet 1 font l'objet de détections par l'appareil de marquage d'événements 25.
On obtient ainsi pour un sujet 60 ayant une foulée de marche normale (Figure 6) et un sujet 90 ayant une foulée de marche pathologique (Figure 7), les accélérations verticale et latérale en fonction du temps.
Ainsi, pour la marche normale, on représente une courbe 40 donnant l'accélération verticale (axe 32, en g) et une courbe 50 donnant l'accélération latérale (axe 33, en g) en fonction du temps (axe 31, en secondes). On identifie sur ces courbes 40 et 50 différentes étapes de la marche et on peut les mettre en relation avec des images synchronisées (prises par une caméra vidéo) du sujet 60 en marche. On repère ainsi respectivement sur les courbes 40 et 50:

- des zones 41 et 51 correspondant à un poser de jambe gauche 90a (image 61),
- des zones 42 et 52 correspondant à un appui bipodal (image 62),
- des zones 43 et 53 correspondant à un lever de jambe droite 90b et à une flexion du genou droit (image 63),
- des zones 44 et 54 correspondant à un appui unipodal vertical de la jambe gauche 90a (image 64), et
- des zones 45 et 55 correspondant à une poussée unipodale de la jambe gauche 90a (image 65).

De manière similaire, les courbes 70 et 80 (Figure 7) représentant respectivement les accélérations verticale et latérale en fonction du temps, on identifie respectivement sur les courbes 70 et 80:

- des zones 71 et 81 correspondant à un poser de jambe droite 90b (image 91),
- des zones 72 et 82 correspondant à un appui bipodal (image 92),
- des zones 73 et 83 correspondant à un lever de jambe gauche 90a et à une flexion du genou gauche (image 93),
- des zones 74 et 84 correspondant à un appui unipodal vertical sur la jambe droite 90b (image 94), et
- des zones 75 et 85 correspondant à une poussée unipodale sur la jambe droite 90b (image 95).

Le procédé d'analyse d'irrégularités de locomotion humaine, fondé sur de tels résultats va maintenant être détaillé. On s'intéresse ainsi à des courbes 100 et 110 ( Figure 6) donnant respectivement les accélérations verticale et latérale (en g, unique axe 34 avec translation de - 1 g pour l'accélération latérale) en fonction du temps (en secondes, axes 31 et 35 respectivement pour les accélérations verticale et latérale et axe 36 de référence). On identifie ainsi des cycles 101-105 sur la courbe 100 d'accélération verticale et des cycles 111-115 sur la courbe 110 (Figure 6) d'accélération latérale, synchronisés et correspondant respectivement à des mouvements des pas gauches et droits.

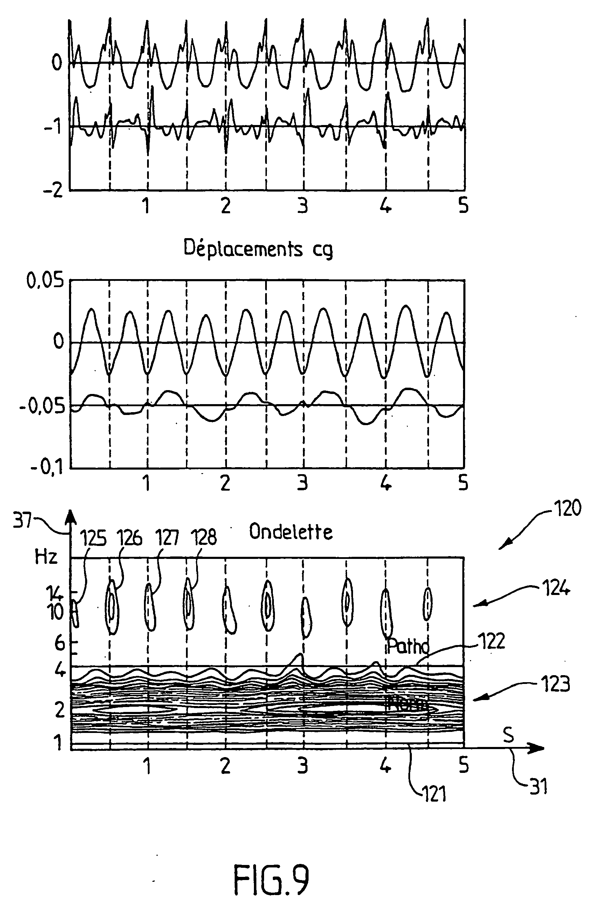

De façon similaire, on obtient par exemple une courbe 140 (Figure 9) donnant l'accélération verticale (axe 32) en fonction du temps (axe 31) pour une marche pathologique, cette courbe 140 admettant également des cycles 141-145.

On soumet l'accélération verticale pour la marche normale et pour la marche pathologique à une transformation en ondelettes continue du type Morlet. Une telle transformation est généralement exprimée sous la forme:

$$C(a,b) = \int_{-\infty}^{+\infty} Acc(t) \cdot \psi\,(at+b)dt,$$

avec:

- la variable t désignant le temps,
- Acc (t) étant le signal d'accélération vertical,
- la fonction $\psi(at+b)$ étant la fonction d'ondelette utilisée, par exemple de Morlet,
- les paramètres a et b de la fonction d'ondelettes étant respectivement les paramètres d'étirement (ou d'échelle) et de translation de l'ondelette,
- les coefficients C (*a, b*) étant les coefficients de la transformée en ondelettes continue.

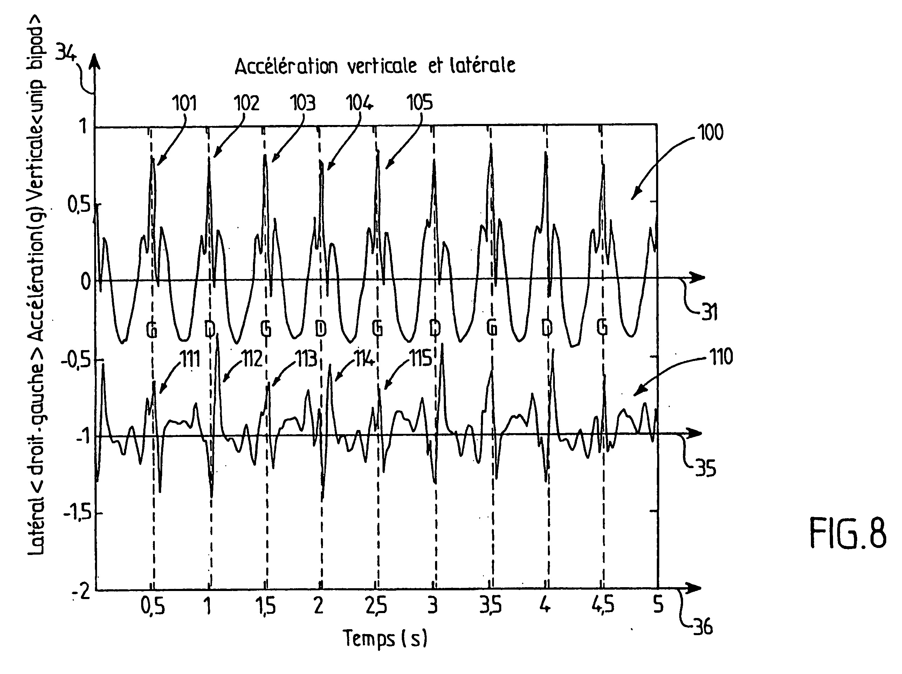

A partir des coefficients C (*a, b*), on construit pour la marche normale une image spectrale 120 (Figures 7 et 8) en trois dimensions, correspondant respectivement au temps, à la fréquence et à l'énergie spectrale. Cette image 120 est représentée dans un plan temps-fréquence (ou temps-échelle) avec une échelle linéaire pour le temps (axe 3, en secondes) et une échelle semi-logarithmique pour la fréquence (axe 37, en Hz). Le module d'énergie ($g^2$) de l'ondelette est représenté par des contours colorés.

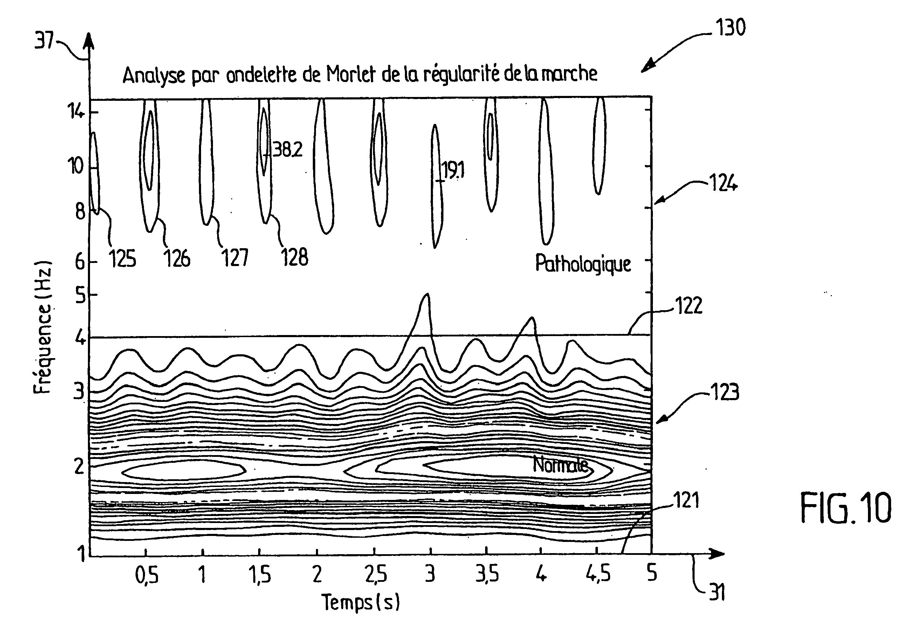

De façon similaire, on obtient pour la marche pathologique correspondant à la Figure 9, une image spectrale 150 de la transformée en ondelettes de Morlet de l'accélération verticale (Figure 10).

La densité d'énergie sur l'agrandissement 130 et sur l'image spectrale 150 est exprimée en accélération ($g^2$).

On définit sur les images spectrales 120 et 150 une bande de basse fréquence ayant des bornes inférieure 121 et supérieure 122, associées respectivement à des valeurs de 1 Hz et de 4 Hz. Dans le cas d'une marche normale, la densité d'énergie du spectre est principalement concentrée dans cette bande. On définit plus précisément une zone normale 123 comprise dans la bande de fréquences et une zone pathologique 124 au-dessus de cette bande.

On observe en effet que pour la marche normale du sujet 60 (Figure 7 et agrandissement 130 de la Figure 8), l'énergie spectrale est essentiellement concentrée dans la zone 123, des pics 125-128 peu élevés et périodiques apparaissant dans la zone pathologique 124 et correspondant aux cycles de marche du sujet 60.

En revanche, sur l'image spectrale 150 associée la marche pathologique du sujet 90, on observe:

- une baisse de l'énergie spectrale totale,

- des pics 155-158 de hautes fréquences ayant une densité énergétique significative dépassant la limite 122 des 4 Hz,
- ces pics 155-158 ayant des altérations de périodicité et de régularité dans le temps et en fréquence.

On peut interpréter les pics 155-158 en énonçant que plus leur forme est graphiquement irrégulière, plus la dynamique et la régularité de la marche sont altérées.

En plus de ces informations graphiques, on calcule des informations quantitatives, comme exposées ci-après. En particulier, on détermine sur les images spectrales 120 et 150, la quantité totale d'énergie spectrale, ainsi que l'énergie spectrale marginale sur l'axe 37 de fréquence, correspondant à une énergie supérieure à la limite 122 des 4 Hz. Les données accumulées sur des cas de référence montrent que pour une marge pathologique, cette énergie marginale est supérieure à 6%.

On obtient ainsi pour la marche normale de l'exemple (Figures 7 et 8) une énergie totale de 22,97 $g^2$ et une énergie marginale représentant 5,3% et pour la marche pathologique (Figure 10), une énergie totale de 2,50 $g^2$ et une énergie marginale représentant 15,4%.

Dans le cas d'une marche très altérée avec des motifs de haute fréquence de forme et d'énergie très variées, on mesure la valeur ponctuelle du module d'énergie sur l'image spectrale.

On complète préférentiellement les informations ainsi obtenues par d'autres techniques d'analyse. En particulier, il est intéressant d'approfondir les informations obtenues par une analyse fréquentielle et un calcul de la symétrie et de la régularité au moyen de la fonction d'autocorrélation, de la manière indiquée dans l'article d'AUVINET et al. cité plus haut. On obtient ainsi pour la marche normale ci-dessus (Figures 6 à 8):

- une fréquence de foulées de 1,00 foulée par seconde,
- une symétrie des foulées de 95,01%, avec une transformée z de Fischer de 183,25, et
- une régularité des foulées de 186,27 (sur 200), avec une transformée z de Fischer de 337,56.

Pour la marche pathologique de l'exemple (Figures 9 et 10), on obtient:

- une fréquence de foulées de 0,88 foulée par seconde (fréquence trop lente),
- une symétrie des foulées de 95,57%, avec une transformation z de Fischer de 189,35, et
- une régularité des foulées de 160,93 (sur 200), avec une transformée z de Fischer de 222,41 (régularité anormale).

On couple ces résultats avec ceux obtenus par les transformations en ondelette. En particulier, on met en relation les pics 125-128 ou 155-158 avec la fréquence des foulées, et leur alternance droite/gauche avec la symétrie, et leur similitude avec la régularité des foulées.

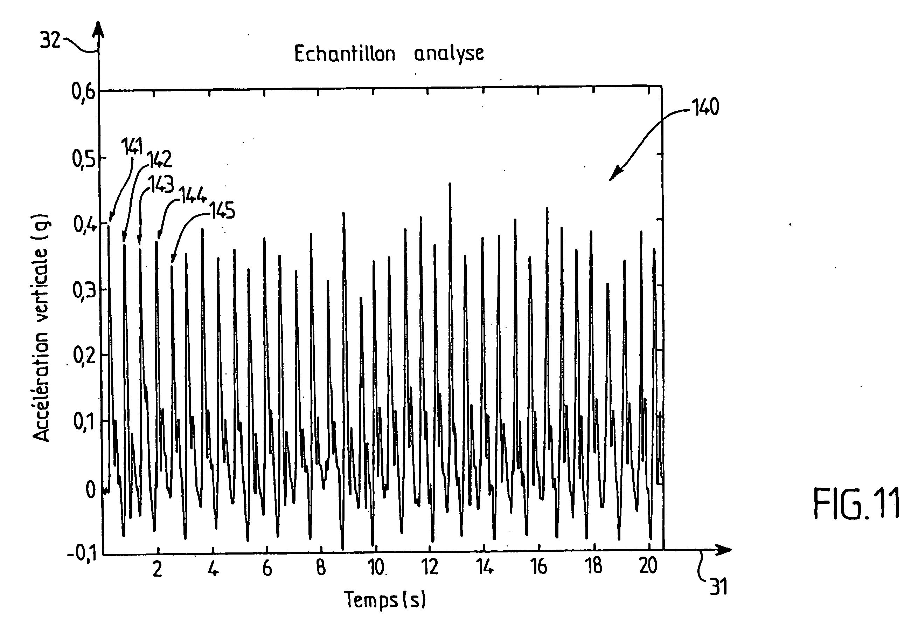

Avantageusement, on complète également ces résultats obtenus par transformation en ondelettes au moyen d'un vectogramme (« papillon frontal »), tel que celui 160 (Figure 11) obtenu pour la marche normale de l'exemple (Figures 6 à 8), donnant l'accélération verticale (axe 32, en g) en fonction de l'accélération latérale (axe 33, en g). On observe sur le papillon frontal 160 des trajectoires cycliques 161-163, qui tendent à être d'autant plus superposées les unes sur les autres que la marche est normale. Dans des cas pathologiques, ces trajectoires cycliques s'éloignent les unes des autres d'une manière identifiable et analysable.

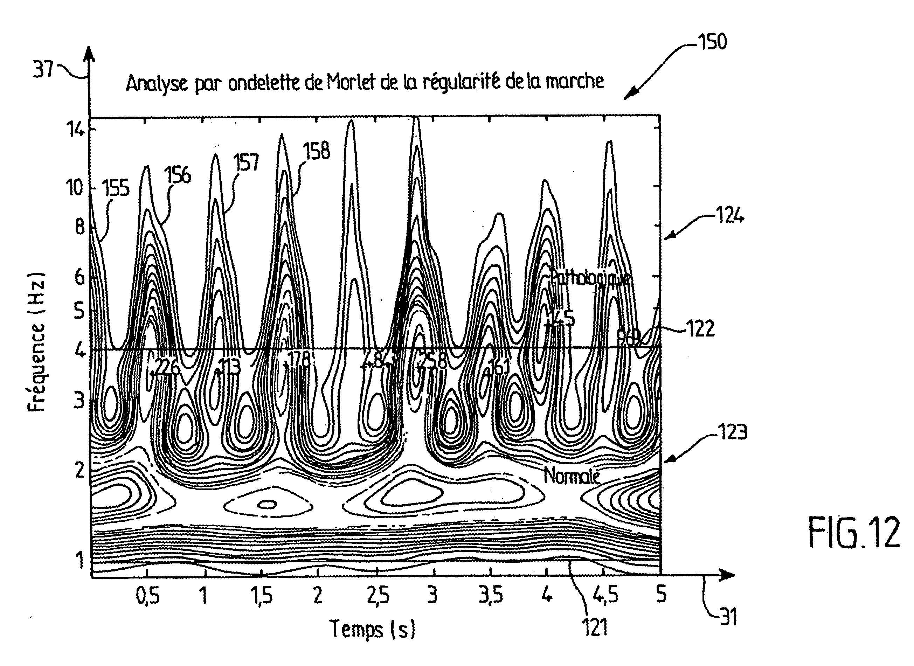

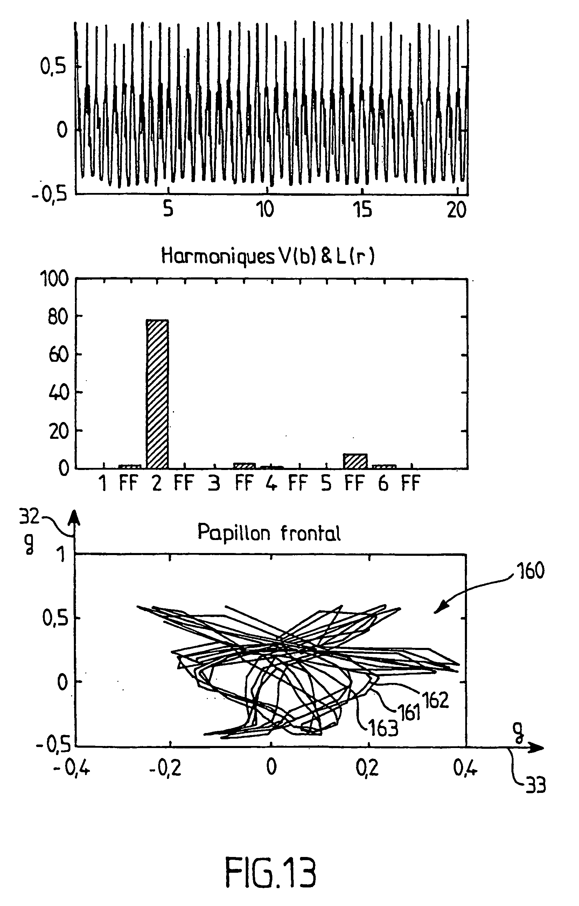

On complète aussi l'analyse par un diagramme des phases, donnant par exemple pour l'accélération verticale de la marche normale (Figure 12) et de la marche pathologique (Figure 13) données en exemple ci-dessus, la dérivée de l'accélération (g.s$^{-1}$, axe 38) en fonction de l'accélération (en g, axe 32). On obtient ainsi des diagrammes 170 et 180 correspondant respectivement aux marches normale et pathologique et ayant respectivement des trajectoires cycliques 171-173 et 181-183.

La marche irrégulière d'un sujet chuteur, par exemple, se manifeste par un régime chaotique, mis en évidence qualitativement par une divergence plus marquée des trajectoires cycliques 181-183.

On quantifie la vitesse de divergence des trajectoires cycliques (ou orbites) du signal au moyen d'au moins un coefficient de Lyapunov. Un tel coefficient évalue la sensibilité d'un système à s'écarter d'un régime stationnaire et périodique à partir d'un point particulier au temps $t_0$ où l'on connaît les caractéristiques du système (conditions initiales).

Le coefficient de Lyapunov moyen $\lambda_m$ de N-1 coefficients de Lyapunov obtenus avec un intervalle d'échantillonnage égal à 1, est classiquement donnée par une formule du type:

$$\lambda_m = \frac{1}{N-1} \cdot \sum_{n=1}^{} ( l_n )$$

avec:

- N étant le nombre de points pris en compte,
- n étant le numéro du point courant, et
- $l_n$ représentant la distance entre le point indexé n appartenant à une orbite et un point voisin le plus proche appartenant à une orbite voisine dans l'espace des phases.

Une méthode pratique de calcul algorithmique de ce coefficient est par exemple exposée dans l'article de WOLF et al., « Determining Lyapunov exponents from a time series », de la revue Physica, 16D, 1985, pp. 285-317.

A titre d'exemple, pour une dimension d'espace des phases égale à 3 et un intervalle d'échantillonnage égal à 3 points, le coefficient de Lyapunov maximal calculé sur le signal accélérométrique vertical est compris entre 0 et 1, ce coefficient étant d'autant plus grand que la pathologie augmente. Ainsi, pour une valeur supérieure à 0,4 la marche est très irrégulière. Ce coefficient quantifie le manque de régularité dynamique des cycles de marche et détecte les fluctuations temporelles et dynamiques de coordination, en relation avec un risque de chute.

On combine l'ensemble des résultats obtenus par ces différentes méthodes, centrées sur l'analyse par ondelette, pour identifier les irrégularités de locomotion et les quantifier.

Les exemples mentionnés, reposant sur les mesures des accélérations verticale et latérale pour une marche, sont particulièrement adaptés à l'identification de claudication ou de dégradation des capacités de personnes âgées, afin de dépister précocement des risques de chute et de mettre en place des mesures de prévention.

Dans d'autres formes de mises en oeuvre, on s'intéresse à d'autres allures que la marche et/ou on utilise d'autres accélérations. Ainsi, par exemple, on analyse une marche sportive en utilisant avantageusement les accélérations verticale et antéro-postérieures du rachis (plan sagittal du sujet, directions 21 et 23). Préférentiellement, on détecte ainsi des fautes techniques d'un athlète de marche sportive, telles qu'une flexion de genou au moment du poser et/ou une rupture simultanée de contact avec le sol des deux pieds de l'athlète.

Dans une autre forme de mise en oeuvre, on analyse une course sportive. Les figures 16 et 17 illustrent l'image spectrale en trois dimensions d'une transformée en ondelettes obtenue respectivement chez un sportif ayant une foulée fluide (figure 16) et chez un sportif ayant une foulée irrégulière (figure 17). Sur la figure 17, on observe sur le tracé d'accélération longitudinal (courbe <fre pro> du haut) et le spectre d'ondelettes correspondant (en bas), un pic de fréquence élevée à 0,2-0,4 sec correspondant à une brusque décélération de freinage au moment du poser du pied gauche. Au temps 0,6-0,8 le poser du pied droit ne présente pas de freinage brutal mais une propulsion plus marquée qu'à gauche. Ces analyses de la foulée permettent de visualiser et caractériser les asymétries de la foulée et de déterminer ainsi les caractéristiques biomécaniques de la foulée d'un athlète. Une comparaison des caractéristiques de la foulée du même athlète analysée à deux moments donnés espacés dans le temps permet d'identifier la présence d'anomalies de la foulée caractérisées par des changements dans ses caractéristiques biomécaniques. Les analyses de la foulée de l'athlète en cause selon le procédé de l'invention permettent aussi de quantifier, et donc de contrôler, la tolérance fonctionnelle d'une affection de l'appareil locomoteur du sportif.

Dans cette autre forme de mise en oeuvre du procédé de l'invention à l'analyse d'une course sportive, la figure 18 montre les courbes d'accélération verticale, antéro-postérieure et latérale en fonction du temps synchronisées avec les images du sujet.

Sur ces courbes, on peut identifier :

- le moment (300) de prise d'appui de la jambe gauche (G);
- le moment (301) de milieu d'appui de la jambe gauche (G);
- le moment (302) de poussée de la jambe gauche (G); et
- le moment (303) de fin d'appui de la jambe gauche (G).

**Revendications**

1. Procédé d'analyse d'irrégularités de locomotion humaine dans lequel on utilise des mesures d'accélération obtenues pendant au moins un déplacement contrôlé à allure stabilisée d'un être humain (1, 60, 90) au moyen d'au moins un accéléromètre mesurant en fonction du temps au moins une accélération selon au moins une direction (21-23), en détectant et en analysant d'éventuelles irrégularités de locomotion à partir de mesures de référence, on soumet lesdites accélérations mesurées à au moins une transformation en ondelettes et on se sert de la transformée en ondelettes obtenue pour détecter et analyser lesdites irrégularités, et **caractérise en ce qu'**on visualise un spectre de ladite transformée en ondelettes en trois dimensions respectivement de temps, de fréquence et de module d'énergie spectrale de la transformée en ondelettes pour détecter et analyser lesdites irrégularités, la transformation consistant en une transformation continue en ondelettes de Morlet **caractérisées par** la formule :

$$\psi (x) = k.\exp(-x^2/2) \cdot \cos (\omega_o x),$$

où k est une constante de normalisation et $\omega_0$ est la pulsation de l'ondelette considérée.

**2.** Procédé d'analyse selon la revendication 1, **caractérisé en ce qu'**on représente le module d'énergie spectrale de la transformée en ondelettes par des contours colorés ou par un gradient de couleurs.

**3.** Procédé d'analyse selon la revendication 2 **caractérisé en ce qu'**on utilise une échelle linéaire pour le temps et logarithmique pour la fréquence.

**4.** Procédé d'analyse selon l'une quelconque des revendications précédentes, **caractérisé en ce que** la transformée en ondelettes ayant une énergie spectrale distribuée dans le temps, on définit une bande de basse fréquence dans laquelle est principalement concentrée l'énergie spectrale pour une locomotion normale et on détecte et/ou on analyse lesdites irrégularités en utilisant l'énergie spectrale en dehors de ladite bande.

**5.** Procédé d'analyse selon la revendication 4, **caractérisé en ce que** ladite bande est comprise entre 1 Hz et 4 Hz.

**6.** Procédé d'analyse selon l'une des revendications 4 ou 5, **caractérisé en ce qu'**on identifie et/ou on analyse lesdites irrégularités en répétant et/ou en étudiant des pics (124-128, 155-158) d'énergie spectrale qui dépassent ladite bande.

**7.** Procédé d'analyse selon l'une quelconque des revendications 4 à 7, **caractérisé en ce qu'**on calcule un degré d'irrégularité de locomotion en rapportant l'énergie spectrale marginale en dehors de ladite bande à l'énergie spectrale totale.

**8.** Procédé d'analyse selon l'une quelconque des revendications précédentes, **caractérisé en ce que** lesdites accélérations sont au moins au nombre de deux et **en ce que** pour détecter et/ou analyser lesdites irrégularités, on utilise aussi au moins un papillon frontal (160) représentant l'intensité d'une des accélérations mesurées en fonction de l'intensité d'une autre des accélérations mesurées.

**9.** Procédé d'analyse selon l'une quelconque des revendications précédentes, **caractérisé en ce que** pour détecter et/ou analyser lesdites irrégularités, on utilise aussi au moins une représentation (170, 180) dans un espace des phases, donnant l'intensité d'au moins une desdites accélérations en fonction de l'intensité de la dérivée temporelle de ladite accélération ou en fonction de ladite accélération décalée dans le temps d'un délai (d) prédéterminé.

**10.** Procédé d'analyse selon la revendication 9, **caractérisé en ce qu'**on calcule au moins un coefficient de Lyapunov de ladite représentation (170, 180) dans l'espace des phases, préférentiellement le coefficient de Lyapunov maximal.

**11.** Procédé d'analyse selon la revendication 10, **caractérisé en ce qu'**on con sidère que se produit un désordre de coordination si le coefficient de Lyapunov maximal est supérieur à 0,4.

**12.** Application du procédé d'analyse selon l'une quelconque des revendications précédentes au domaine médical, en particulier pour la détection précoce de désordres neuro-moteurs de sujets âgés.

**13.** Application du procédé d'analyse selon l'une quelconque des revendications 1 à 11 au domaine sportif, en particulier pour la détection de fautes techniques de marcheurs ou de coureurs sportifs.

**14.** Application du procédé d'analyse selon l'une quelconque des revendications 1 à 11 pour la détection des particularités de foulée de l'athlète pendant une période de course.

**15.** Application du procédé d'analyse selon l'une quelconque des revendications 1 à 11, pour mesurer et quantifier les contraintes biomécaniques liées à une affection de l'appareil locomoteur.

**Claims**

1. Method of analysis for human locomotion irregularities, wherein acceleration measurements obtained during at least one motion controlled at a stabilised gait of a human being (1,60,90) are used, through at least one accelerometer measuring on a time base at least one acceleration according to at least one direction (21-23), by detecting and analysing any locomotion irregularities from reference measurements, said measured accelerations are submitted to at least one wavelet transformation and the resulting wavelet transform is used to detect and analyse said irregularities, and **characterised in that** the spectrum of said wavelet transform is visualised in three dimensions, respectively, of time, frequency and spectral energy module of the wavelet transform to detect and analyse said irregularities, the transformation consisting of a Morlet Wavelet continuous transformation of formula :

$$\psi(X) = k.\exp(-x^2/2) . \cos(\omega_o X),$$

wherein k is a normalisation constant and $\omega_o$ is the pulse of the walvelet being considered.

2. Method of analysis according to claim 1, **characterized in that** the spectral energy module of the wavelet transform is represented by coloured contours or a colour gradient.

3. Method of analysis according to claim 2, **characterized in that** a linear scale for time and a logarithmic scale for frequency are used.

4. Method of analysis according to any one of the preceding claims, **characterized in that** the wavelet transform having a time-dependant spectral energy, a low frequency band is defined in which the spectral energy for a regular locomotion is mainly concentrated and said irregularities are detected and/or analysed by using the spectral energy outside such band.

5. Method of analysis according to claim 4, **characterized in that** the said band is comprised between 1 Hz and 4 Hz.

6. Method of analysis according to any one of claims 4 or 5, **characterized in that** said irregularities are identified and/or analysed by locating and/or studying the spectral energy peaks (124-128, 155-158) exceeding said band.

7. Method of analysis according to any one of claims 4 to 6, **characterized in that** a locomotion irregularity degree is calculated by reporting the margin spectral energy outside said band to the total spectral energy.

8. Method of analysis according to any one of the preceding claims, **characterized in that** said accelerations are at least in a number of two and **in that**, to detect and/or analyse the said irregularities, at least one front butterfly (160) is also used representing the intensity of one of the measured accelerations depending on the intensity of another of the measured accelerations.

9. Method of analysis according to any one of the preceding claims, **characterized in that**, to detect and/or analyse the said irregularities, at least one representation (170, 180) in a phase space is also used, giving the intensity of at least one of said accelerations depending on the intensity of the time derivative of said acceleration or depending on said acceleration time shifted with a predetermined time (d).

10. Method of analysis according to claim 9, **characterized in that** at least one Lyapunov coefficient of said representation (170,180) in the phase space, preferably the maximum Lyapunov coefficient, is calculated.

11. Method of analysis according to claim 10, **characterized in that** it is considered that a co-ordination disorder occurs if the maximum Lyapunov coefficient is higher than 0.4.

12. Application of the method of analysis according to any one of the preceding claims for the medical field, in particular for the early detection of neuro-motor disorders of elderly people.

13. Application of the method of analysis according to any one of claims 1 to 11 for the sport field, in particular for detecting technical defects with sport walkers or runners.

**14.** Application of the method of analysis according to any one of claims 1 to 11 for detecting athlete's stride particularities during a race period.

**15.** Application of the method of analysis according to any one of claims 1 to 11 for measuring and quantifying the biomechanical constraints associated to a disease of the locomotive apparatus.

**Patentansprüche**

**1.** Verfahren zur Analyse von Irregularitäten der menschlichen Bewegung, bei dem Beschleunigungsmessungen verwendet werden, welche während mindestens einer kontrollierten Bewegung eines menschlichen Wesens (1, 60, 90) bei stabilem Tempo mittels mindestens eines Beschleunigungsmessers, der in Abhängigkeit von der Zeit mindestens eine Beschleunigung in mindestens einer Richtung (21-23) misst, durch Erkennen und Analysieren eventueller Irregularitäten der Bewegung ausgehend von Referenzmessungen erhalten werden, wobei die gemessenen Beschleunigungen mindestens einer Wavelet-Transformation unterzogen werden, und wobei die erhaltene Wavelet-Transformation zum Erkennen und Analysieren der Irregularitäten verwendet wird, und wobei ein Spektrum der genannten Wavelet-Transformation in drei Dimensionen, nämlich der Zeit, der Frequenz und dem Spektralenergiemodul der Wavelet-Transformation visualisiert wird, um diese Irregularitäten zu erkennen und zu analysieren, **dadurch gekennzeichnet, dass** die Transformation eine kontinuierliche Morlet-Wavelet-Transformation ist, welche durch die Formel:

$$\psi(x) = k.\exp(-x^2/2) \cdot \cos(\omega_0 x)$$

angegeben ist, wobei k eine Normalisierungskonstante und $\omega_0$ die Pulsation des betreffenden Wavelets ist.

**2.** Analyseverfahren nach Anspruch 1, **dadurch gekennzeichnet, dass** das Spektralenergiemodul der Wavelet-Transformation durch farbige Konturen oder einen Farbgradienten wiedergegeben wird.

**3.** Analyseverfahren nach Anspruch 2, **dadurch gekennzeichnet, dass** eine lineare Skala für die Zeit und eine logarithmische Skala für die Frequenz verwendet wird.

**4.** Analyseverfahren nach einem der vorhergehenden Ansprüche, **dadurch gekennzeichnet, dass**, da die Wavelet-Transformation eine über die Zeit verteilte Spektralenergie hat, ein Band mit tiefer Frequenz definiert wird, in welchem die Spektralenergie bei einer normalen Bewegung hauptsächlich konzentriert ist, und die Irregularitäten unter Verwendung der außerhalb dieses Bandes liegenden Spektralenergie erkannt und/oder analysiert werden.

**5.** Analyseverfahren nach Anspruch 4, **dadurch gekennzeichnet, dass** das Band zwischen 1 Hz und 4 Hz liegt.

**6.** Analyseverfahren nach Anspruch 4 oder 5, **dadurch gekennzeichnet, dass** die Irregularitäten erkannt und/oder analysiert werden, indem Spitzen (124-128, 155-158) der Spektralenergie, welche das Band übersteigen, wiederholt und/oder untersucht werden.

**7.** Analyseverfahren nach einem der Ansprüche 4 bis 7, **dadurch gekennzeichnet, dass** ein Grad der Irregularität der Bewegung berechnet wird, indem die außerhalb des Bandes liegende Rand-Spektralenergie in Bezug zu der Gesamtspektralenergie setzt.

**8.** Analyseverfahren nach einem der vorhergehenden Ansprüche, **dadurch gekennzeichnet, dass** die Anzahl der Beschleunigungen mindestens zwei beträgt, und dass zum Erkennen und/oder Analysieren der Unregelmäßigkeiten eine frontale Schmetterlingskurve (160) verwendet wird, welches die Intensität einer der gemessenen Beschleunigungen als Funktion der Intensität einer anderen der gemessenen Beschleunigungen wiedergibt.

**9.** Analyseverfahren nach einem der vorhergehenden Ansprüche, **dadurch gekennzeichnet, dass** zum Erkennen und/oder Analysieren der Irregularitäten ferner mindestens eine Darstellung (170, 180) in einem Phasenraum verwendet wird, welche die Intensität mindestens einer der Beschleunigungen als Funktion der Intensität der zeitlichen Ableitung der Beschleunigung oder als Funktion dieser jedoch um eine bestimmte Verzögerung (d) zeitlich versetzten Beschleunigung wiedergibt.

10. Analyseverfahren nach Anspruch 9, **dadurch gekennzeichnet, dass** mindestens ein Lyapunov-Koeffizient der genannten Darstellung (170, 180) im Phasenraum berechnet wird, vorzugsweise der maximale Lyapunov-Koeffizient.

11. Analyseverfahren nach Anspruch 10, **dadurch gekennzeichnet, dass** von einer Koordinationsstörung ausgegangen wird, wenn der maximale Lyapunov-Koeffizient größer als 0,4 ist.

12. Anwendung des Analyseverfahrens nach einem der vorhergehenden Ansprüche im medizinischen Bereich, insbesondere zur frühzeitigen Erkennung neuromotorischer Störungen älterer Personen.

13. Anwendung des Analyseverfahrens nach einem der Ansprüche 1-11 im sportlichen Bereich, insbesondere zur Erkennung von technischen Fehlern von Sportgehern oder -läufern.

14. Anwendung des Analyseverfahrens nach einem der Ansprüche 1-11 zur Erkennung von Besonderheiten des Schritts eines Athleten während eines Zeitraums eines Laufs.

15. Anwendung des Analyseverfahrens nach einem der Ansprüche 1-11 zum Messen und Quantifizieren der mit einer Beeinträchtigung des Bewegungsapparats einhergehenden biomechanischen Belastungen.

FIG.1

FIG.2

FIG.3

FIG.4

FIG.5

FIG.6

FIG.7

FIG.8

Accélération verticale et latérale

100

31

110

35

36

Temps (s)

101  102  103  104  105

111  112  113  114  115

34

Lateral < droit-gauche > Accélération (g) Vertical < droit-gauche > unip bipod >

Déplacements cg

Ondelette

FIG.9

Analyse par ondelette de Morlet de la régularité de la marche

FIG.10

Echantillon analyse

FIG.11

Analyse par ondelette de Morlet de la régularité de la marche

FIG.12

EP 1 261 279 B1

FIG.13

Diagramme des phases d'accélération verticale

FIG.14

Diagramme des phases d'accélération verticale

FIGURE 16

ACCELERATION VERTICALE, LONGITUDINALE ET LATERALE

ONDELETTES DES MOUVEMENTS VERTICAUX

ONDELETTES DES MOUVEMENTS LONGITUDINAUX

FIGURE 17

FIGURE 18

EP 1 261 279 B1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Analyse de la marche humaine dans la pratique hospitalière par une méthode accélérométrique. **BERNARD AUVINET ; DENIS CHALEIL ; ERIC BARREY.** REV. RHUM. 1999, vol. 66, 447-457389-397

- **SEKINE et al.** Classification of accélération waveform in a continuous walking record. *Proceedings of the 20th Annual conference of the IEEE in Medicine and Biology Society,* 1988, vol. 20 (3), 1523-1526
- **WOLF et al.** Determining Lyapunov exponents from a time series. *Physica,* 1985, vol. 16D, 285-317